(19) Europäisches Patentamt
European Patent Office
Office européen des brevets

(11) Publication number: **0 364 033 A2**

# (12) EUROPEAN PATENT APPLICATION

(21) Application number: 89202517.2

(51) Int. Cl.5: C07F 7/18

(22) Date of filing: 06.10.89

(30) Priority: 12.10.88 US 256539

(43) Date of publication of application: 18.04.90 Bulletin 90/16

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Bender, Dean R.
40 Monterey Drive
Hazlet New Jersey 07730(US)
Inventor: Demarco, Anthony M.
RD No.1 Box 265
Glen Gardner New Jersey 08826(US)
Inventor: Melillo, David G.
2637 Crest Lane
Scotch Plains New Jersey 07070(US)
Inventor: Shinkai, Ichiro
1101 Prospect Street
Westfield New Jersey 07090(US)

(74) Representative: Hesketh, Alan, Dr. et al
European Patent Department Merck & Co., Inc. Terlings Park Eastwick Road
Harlow Essex, CM20 2QR(GB)

## (54) Stereoselective process for producing 1-beta-methylcarbapenem antibiotic intermediates.

(57) A methylation process for producing an alpha methylated azetidinone alkyl ester in high yield by the use of a sodium bis(trialkylsilyl)amide. The alpha-methyl ester can be isomerized to the corresponding desired beta-methyl isomer. The beta-methyl isomer can also be directly produced by the use of lithium dialkylamide and trialkylaluminum. The beta-methyl compound is an intermediate in the synthesis of 1-beta-methyl-carbapenem antibacterial agents.

EP 0 364 033 A2

# STEREOSELECTIVE PROCESS FOR PRODUCING 1-BETA-METHYLCARBAPENEM ANTIBIOTIC INTERMEDIATES

## BACKGROUND OF THE INVENTION

### 1. Field of the Invention

This invention relates to a process for producing 1-betamethyl carbapenem antibiotic intermediates involving the high yield synthesis of an alpha-methylated azetidinone alkyl carboxylic ester which can be isomerized to the beta-methyl isomer, or the direct synthesis of the beta-methyl isomer utilizing a trialkylaluminum.

### 2. Brief Description of Disclosures in the Art

Since the discovery of thienamycin,

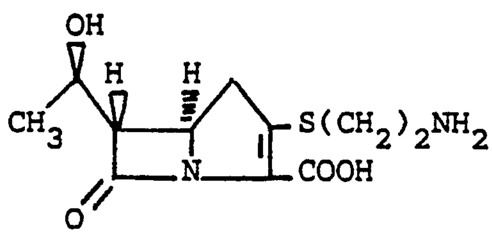

an extremely potent broad spectrum antibiotic, disclosed and claimed in commonly assigned U.S.P. 3,950,357, a large amount of research activity has been conducted in the medicinal chemistry area for other active analogs not having its associated deficiencies, i.e. chemical instability at high concentration and susceptibility to renal Dehydropeptidase I.

In addition to the N-formimidoyl derivative of thienamycin, disclosed and claimed in commonly assigned U.S.P. 4,194,047, among some of the more promising analogs that have been developed are the 1-betamethyl compounds of the structure, i.e.,

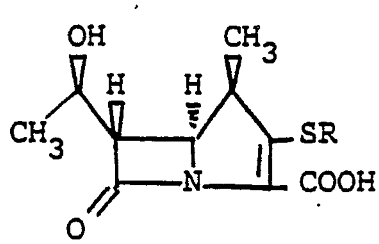

in which the 1 methyl group is in the beta configuration and R is a radical known in the antibiotic art.

Synthesis of the above 1-beta methyl analogs is desired in large quantities for pharmacological evaluation and requires a process which is environmentally safe for the introduction of the beta-methyl substituent in a manner designed to yield a high percentage of the beta-methyl intermediate prior to ring closure to the carbapenem system.

A published procedure by Shih et al. in Heterocycles, Vol. 21, No. 1, pp. 29-40 (1984) describes a process for alkylating the 4-alkyl side chain of certain azetidin-2-ones in the presence of hexamethylphosphoramide (HMPA) as a cosolvent, to produce a mixture of the alpha-and beta-methyl isomers in about a 4:1 molar ratio. The reference also describes an epimerization process employing HMPA as a cosolvent resulting in a 1:1 beta/alpha methyl isomer ratio.

Desirably, a large scale should achieve higher beta/alpha isomeric molar ratios than this and the use of hexamethyl-

phosphoramide should be avoided due to its known carcinogenicity.

It is therefore an object of this invention to provide a process for producing intermediates useful in making 1-betamethylcarbapenem antibiotics. It is further an object of this invention to provide a process for producing intermediates in high yield having the necessary 1-beta methyl stereochemistry, prior to ring closure to the carbapenem ring system, in which the products contain the beta methyl/alpha methyl isomers in a molar ratio greater than one. These and further objects of the invention will become obvious from the accompanying disclosure as set forth herein.

## SUMMARY OF THE INVENTION

It has been found that by treating a compound of the structure:

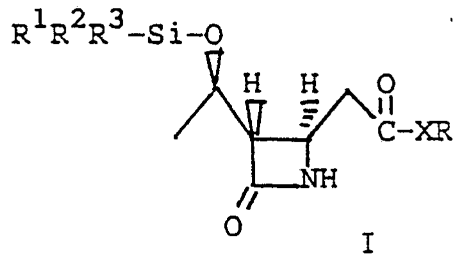

I

with a sodium bis(trialkylsilyl)amide followed by a methylating agent, good yields without the need of a cosolvent of the alpha methyl isomer are obtained Use of the sodium enolate, as contrasted with the lithium or potassium enolates, allows both high overall yields and high throughput concentrations in a large scale process. The Li enolate gives good concentrations, e g. 0.2 M, but lower yields, e.g. 60-65%, while the K enolate gives high yields, 80-85 + %, but requires low concentrations due to poor solubility, e.g. 0.06 M. The sodium enolate, by contrast, gives high concentrations 0.2 M, and high yields e.g. 80-85%. The resulting alpha isomer can be isomerized to the beta-methyl isomer, useful in the synthesis of 1-β-methyl-carbapenem antibiotics, by the procedure described above in the Shih reference, and in EP-A 241,200 hereby incorporated by reference for this particular purpose. This process is referred to as the "indirect" process.

Also, it has been found that by forming the lithium enolate of I at low temperature, then forming the trialkylaluminum complex, the complex can be directly methylated to produce in good yield the desired "beta" isomer

In accordance with the invention there is provided a process comprising the steps of

(a) contacting the compound:

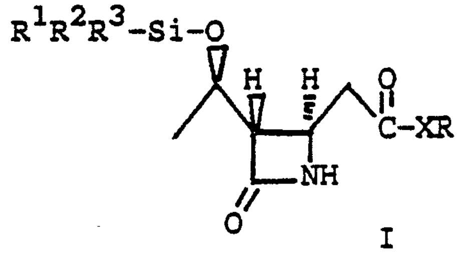

I

wherein X is -O- or -S-; R is $C_1$-$C_8$ linear or branched alkyl, $C_6$-$C_8$ aryl or a carboxylic acid protecting group; and $R^1$, $R^2$, $R^3$ are selected from $C_1$-$C_8$ linear or branched alkyl, $C_6$-$C_8$ aryl; with at least two equivalents proportionately thereto of a sodium metal bis(trialkylsilyl)amide, in an anhydrous solvent therefor, excluding hexamethylphosphoramide, in an oxygen-free, moisture-free atmosphere;

(b) contacting the resulting mixture from step (a) with a methylating agent to produce a compound of the structure:

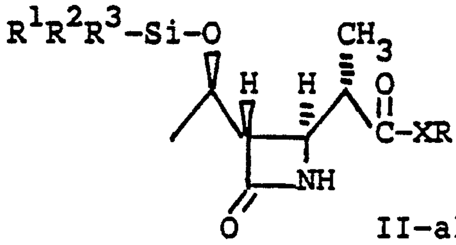

II-alpha

Further there is provided a process comprising the steps of:

(a) contacting the compound:

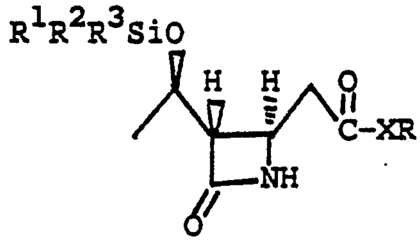

I

wherein X is -O- or -S-; R is $C_1$-$C_8$ linear or branched alkyl, $C_6$-$C_8$ aryl, or a carboxylic acid protecting group; and $R^1$, $R^2$, $R^3$ are selected from $C_1$-$C_8$ linear or branched alkyl, $C_6$-$C_8$ aryl; with a lithium metal amide in an anhydrous solvent therefor, excluding hexamethylphosphoramide, in an oxygen-free, moisture-free atmosphere;

(b) contacting the resulting mixture from step (a) with a tri-$C_1$-$C_6$ alkylaluminum;

(c) contacting the resulting mixture from step (b) with a methylating agent to produce a compound of the structure:

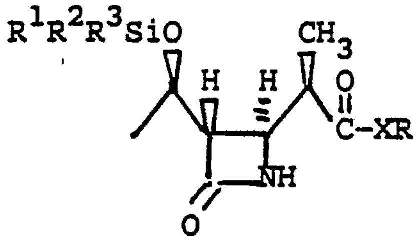

II-beta

## DESCRIPTION OF THE INVENTION AND PREFERRED EMBODIMENTS

The overall subject invention can be easily understood from the following illustrated reaction scheme:

Reaction Scheme

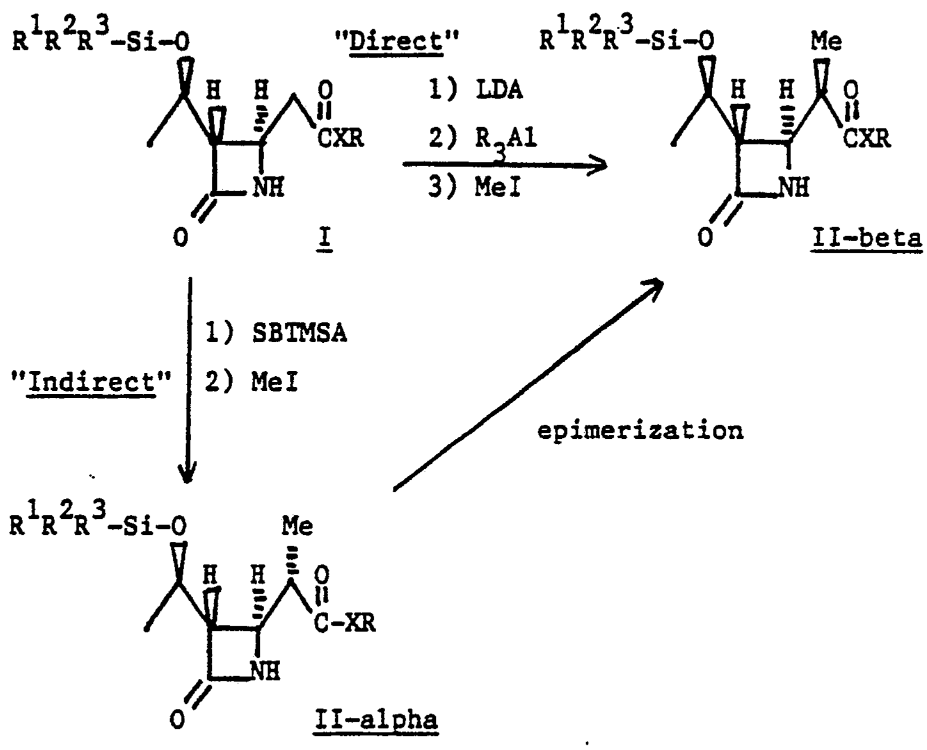


The above reaction scheme illustrates the overall process of producing desired 1-beta methylcarbapenem intermediates II-beta involving the "indirect" process of methylating the azetidinone ester I - (wherein X is O) to form the alpha methylated azetidinone II-alpha, which can then be isomerized to the beta-form; and the "direct" process which produces the beta isomer in good yield directly without proceeding through the alpha form.

The "indirect" method for methylating the sodium enolate of the azetidinone ester I is a novel modification of the above referred to method of Shih et al., hereby incorporated by reference, which involves a similar procedure as described but eliminates the use of hexamethylphosphoramide as a cosolvent, and uses a different metal amide base, to produce in significantly higher yield and at a reaction concentration suitable for high throughput production a mixture of the alpha- and beta-methyl isomers in about a 98:2 molar ratio.

The R radical in the ester group -XR, being -OR or -SR, in Structure I is a linear or branched $C_1$-$C_8$ alkyl, $C_6$-$C_8$ or carboxylic acid protecting group. Representative examples include methyl, ethyl, isopropyl, propyl, butyl, iso-butyl sec-butyl, benzyl, phenyl, phenethyl, p-nitrophenyl, p-nitrobenzyl, and the like. Preferred is methyl.

The R groups on the silicon protecting group are selected from $C_1$-$C_8$ linear or branched alkyl, or $C_6$-$C_8$ aryl. Representative R groups include methyl, ethyl, propyl, isopropyl, sec-butyl, n-butyl, isobutyl, t-butyl, benzyl, phenyl, phenethyl, p-tolyl, meta-tolyl, 3,5-dimethylphenyl, and the like. A preferred silyl group is dimethyl t-butylsilyl.

The sodium metal amide used in the indirect process to form the dianion is a sodium metal bis(tri-$C_1$-$C_8$-alkylsilyl)amide in which the alkyl substituents on the amide may be chosen from methyl, ethyl, propyl, isopropyl, n-butyl, t-butyl, isobutyl, secondary butyl, and cycloalkyl such as cyclohexyl. Preferred is where both groups are the same and particularly preferred being trimethylsilyl. A preferred sodium metal amide to form the dianion in step (a) is sodium bis(trimethylsilyl)amide also referred to herein as "SBTMSA". The sodium metal amide may be purchased in commercially available pre-made form or can be prepared in situ from sodium and amines by known procedures in the art.

The methylating agent used is conventional and can be methyl iodide, methyl bromide, dimethyl sulfate, and the like.

The solvent used in either process is generally an anhydrous organic ether in which the ether can be cyclic or acyclic and can contain $C_2$-$C_{10}$ carbon atoms. Representative examples of such solvents include tetrahydrofuran (THF), dioxane, glyme, diglyme, methyl t-butylether, diethyl ether and the like. A preferred

ether for use in the process is THF.

The use of a solvent in the process is conducted in the absence of hexamethylphosphoramide which in fact is a carcinogenic agent and to be avoided in commercial processes.

Both methylations are conducted in an oxygen-free, moisture-free atmosphere to avoid contamination or side reactions from occurring with the sodium metal amide and its dianion products and solution.

Generally, the compound of structure I is added in solution to a solution of the sodium metal amide and in a solvent described above at a temperature at a range of about -30° to about -80° C with stirring. The reverse mode of addition can also be carried out. Preferred is where the structure I is added to the amide. After the reaction contents have been mixed, to ensure complete dianion formation, they are gently stirred for several hours, e.g. from about 1 to 4 hours. The methylation is carried out at the above temperature range, -30 to 80° C, usually with a slight excess of methylating agent, e.g. methyl iodide and bromide, preferably iodide. The methylation mixture is generally allowed to stir under a nitrogen inert atmosphere and then quenched with a protic organic acid, such as acetic acid. Then an organic solvent/water mixture, e.g. isopropyl acetate/water is added, and then the solvent of the organic phase is changed to heptane to completely precipitate the methylated isomers by simple crystallization. Yields of the alpha isomer are high and in a high alpha/beta molar ratio of about 95-98:5-2.

The resulting alpha isomer can be subjected to an epimerization process and can be converted readily and very conveniently to the beta methyl isomer under relatively mild and convenient reaction conditions, as described in EP-A-241,200 hereby incorporated by reference. The overall epimerization process consists generally of three steps involving (a) formation of a dianion by means of an alkali metal amide, followed by step (b) in which the dianion is contacted with a metallic cation containing salt, a P-H or S-H organic protic organic acid, a Sn or Pb organometallic hydride, or a trialkyl borane reagent and in step (c) contacting this resulting mixture with an OH organic protic acid or mineral acid to convert the alpha-methyl isomer substantially in good yield to the beta-methyl isomer. Generally, the reaction results in a mixture of beta- and alpha-methyl isomers which is substantially enriched with respect to the beta isomer, which is then subjected to a separation process to obtain pure beta-methyl isomer.

The "direct" methylation process involves reacting the lithium dianion derived from I, described hereinabove, with a trialkyl aluminum and then methyl iodide.

The lithium metal amide used in step (a) to form the dianion is a lithium metal di-$C_1$-$C_8$-alkyl amide in which alkyl substituents on the amide may be chosen from methyl, ethyl, propyl, isopropyl, n-butyl, t-butyl, isobutyl, secondary butyl, and cycloalkyl such as cyclohexyl. Preferred is where both groups are the same and particularly preferred being isopropyl, i.e., lithium diisopropylamide. The lithium metal amide may be purchased in commercially available pre-made form or can be prepared in situ from alkyl lithium and amines by known procedures in the art.

The solvent used in step (a) and throughout steps (b) and (c) is generally an anhydrous organic ether in which the ether can be cyclic or acyclic and can contain $C_2$-$C_{10}$ carbon atoms. Representative examples of such solvents include tetrahydrofuran (THF), dioxane, glyme, diglyme, methyl t-butylether, diethyl ether and the like. A preferred ether for use in the process is THF.

The use of a solvent in step (a) is conducted in the absence of hexamethylphosphoramide which in fact is a carcinogenic agent and to be avoided in commercial processes.

The reaction of step (a) is conducted in an oxygen-free, moisture-free atmosphere to avoid contamination or side reactions from occurring with the alkali metal amide and its dianion products and solution.

Generally, the compound of structure I is added in solution to a solution of the amide and in a solvent described above at a temperature at a range of about -30° to about -80° C with stirring. The reverse mode of addition can also be carried out. Preferred is where the structure I is added to the amide. After the reaction contents have been mixed, to ensure complete dianion formation, they are gently stirred for several hours, e.g. from about 1 to 4 hours.

The second step (b) of the process involves reacting the lithium dianion with a trialkylaluminum reagent.

Trialkyl aluminum agents are selected from $C_1$-$C_8$ linear or branched alkyl aluminum compounds. Representative alkyls may be selected from methyl, ethyl, propyl, butyl, isopropyl, isobutyl, secondary butyl, tertiary butyl, cycloalkyl such as cyclohexyl and benzyl. Representative examples include trimethylaluminum, triethylaluminum, tripropylaluminum, triisobutylaluminum and tri-sec-butylaluminum. A preferred aluminum agent which can be utilized in the invention process is triethylaluminum.

In the direct methylation, the temperature of the addition of the aluminum reagent to the solution resulting from step (a) is normally conducted in the range of about -40° to -80° C. The reaction solution is then stirred for a period of 0.3 to 4 hours at -40° to -80° C.

The methylating reagent is conventional, e.g. methyl iodide or methyl bromide, and the conditions are substantially the same as described for the "indirect process" described above.

Following the contacting of the lithium trialkyl aluminum complex of I with the methylating agent, the resulting mixture is contacted with an -OH organic protic acid or mineral acid, thus forming a mixture of the alpha- and beta-methyl isomers.

The -OH containing organic protic acids and mineral acids include protonated oxygen and nitrogen compounds, respectively. Representative examples of said protic acids include alkyl and aryl carboxylic acids, protonated amines and strong mineral acids. Representative protic acids include aqueous ammonium chloride, sulfuric acid, hydrochloric acid, acetic acid, formic acid, and the like. A preferred protic acid in the process is acetic acid.

The addition of the protic acid is conducted in the range of -20° to -100° C and the addition can be made either by adding the acid to the resulting mixture from step (b) or vice versa, preferred is the former mode.

The concentration of Structure I, used in the direct methylation is preferably about 0.14 M, but no more than about 0.3 molar to inhibit precipitation of the enolate salt. Larger concentrations can be used but will result in a two-phase system more difficult to work with.

In the"direct" process, proportionately for every mole of starting structure I used:

a) 2 or more moles of the lithium metal amide are employed prepared in situ or commercially pre made;

b) if prepared in situ, 2 or more moles of alkyl lithium, eg. butyl lithium, are used to generate the lithium dialkylamide, in which an equal or slightly smaller amount proportionately than the amine is used thus generating at least 2 equivalents of the lithium alkylamide per equivalent of I;

c) 1 or more moles of trialkyl aluminum, are used, and preferably two;

d) at least two moles of protic acid are used in step (c) and an amount at least equal to the amount of butyl lithium used. Sufficient acid should be employed in step (c) to ensure neutral workup conditions.

The mixture resulting from the "direct" methylation reaction generally results in a beta to alpha isomer mixture in which the beta to alpha isomer ratio is greater than 70/30. Generally, the ratio is in the range of 75/25 and above and can be obtained in the range of 75/25 to 85/15.

When using the indirect process, the alpha to beta molar ratio as high as 98:2 can be obtained after simple crystallization.

Separation techniques are performed on the mixture resulting from the "direct" process, by conventional techniques including high pressure liquid chromatography (HPLC), and the like, and also a new technique, i.e. cyclic selective dissolution (SWISH), described in copending application Serial No. 134,589, (Case 17392CA) incorporated by reference herein, allow the separation of pure 5-beta-methyl azetidinone ester II-βeta and the substantially pure alpha methyl isomeric mixture II-alpha.

The following examples are illustrative of the subject invention and should not be construed as being limitations on the scope or spirit thereof.

## EXAMPLE 1

### Preparation of the α-Methyl Derivative

A solution of $NaN(SiMe_3)_2$ in THF (1M, 764 mL) was diluted with THF (300 mL) and cooled to -80° C. To the resulting suspension was added a solution of (3S,4R)-3-[(1R)-1-t-butyldimethyl-silyloxyethyl]-4-methoxycarbonylmethylazetidin-2-one (100.1 g) in THF (500 mL). The resulting solution was aged at -78° C for 30 min, then at -50° C for 1.5 hours, and then cooled to -80° C. Methyl iodide (37.2 mL) was then added and the resulting solution aged at -80° C for 1 hour, then at -50° C for 0.5 hour, and then cooled to -75° C. A solution of acetic acid (100 mL) in THF (200 mL) was added while maintaining the temperature at -75° C to -60° C. The resulting mixture was warmed to -20° C. Water (1L) and isopropyl acetate (1L) were added with agitation. The separated organic layer was washed sequentially with 5% aq. $NaHSO_3$ (800 mL), 5% aq. $NaHCO_3$ (800 mL) and water (800 mL). The washed organic layer was then concentrated to a thick slurry (200 mL) and sequentially flushed with isopropyl acetate (200 mL) and heptane (200 mL). Additional heptane (450 mL) was added. The mixture was heated to dissolve all solids, and then was slowly cooled to 10° C and aged for about 30 minutes. Filtration afforded 85.4g (82% yield) of methylated material having an isomer composition (α/β) of 98/2.

## EXAMPLE 2

Preparation of mixture containing predominantly the β-Methyl derivative

A solution of THF (2 mL) and diisopropyl amine (0.25 mL) was cooled to -40° C, a butyllithium/hexane solution (2.4 M, 0.67 mL, 1.6 mmol) was added, and the resulting solution was cooled to -75° C. To this was added a solution of (3S,4R)-3-[(1R)-1-t-butyldimethylsilyloxyethyl] 4 methoxycarbonylmethyl azetidin-2-one (482 mg, 1.6 mmol) in THF (2.5 mL). After aging the resulting solution at -75° C for 20 min, a hexane solution of triethylaluminum (1.0 M, 1.6 mL) was added, and the solution was aged at -75° C for 35 min. During the aging, a solution of lithium diisopropylamide (1.6 mmole) was prepared as described above using THF (1.5 mL), diisopropylamine (0.25 mL) and butyllithium/hexane (2.4 M, 0.67 mL). This LDA solution was then added to the reaction solution, and the resulting solution aged at -75° C for 68 min. Additional triethylaluminum (1.0 M hexane solution, 1.6 mL) was then added and the reaction solution further aged (-75° C/40 min). Methyl iodide (1.0 mL) was added, and the resulting mixture was aged at -75° C for 1.7 h. A small portion of the mixture was quenched by transfer via a cooled cannula to a solution of HOAc in THF at -70° C. The resulting quench mixture was warmed to -25° C, then water and ethyl acetate were added with agitation. The separated organic layer was assayed by HPLC and compared with standards of authentic material. Isomer amounts thus measured were β-methyl, 36 mg; α-methyl, 9 mg (β/α = 80/20).

The remainder of the reaction mixture was warmed to -50° C and aged at that temperature for 1.25 hours. Quenching and assaying as described above showed the presence of β-methyl, 192 mg; α-methyl, 63 mg (β/α = 75/25). Total yield (α + β from both quenches) = 59.5%. Total yield of β = 45%.

**Claims**

1. A process comprising the steps of:
(a) contacting the compound:

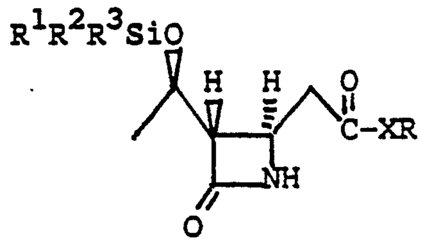

I

wherein X is -O- or -S-; R is $C_1$-$C_8$ linear or branched alkyl, $C_6$-$C_8$ aryl, or a carboxylic acid protecting group; and $R^1$, $R^2$, $R^3$ are selected from $C_1$-$C_8$ linear or branched alkyl, $C_6$-$C_8$ aryl; with at least two equivalents proportionately thereto of a sodium metal bis(trialkylsilyl)amide in an anhydrous solvent therefor, excluding hexamethylphosphoramide, in an oxygen-free, moisture-free atmosphere;

(b) contacting the resulting mixture from step (a) with a methylating agent to produce a compound of the structure:

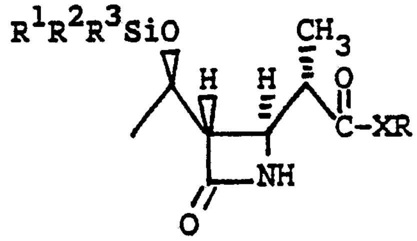

II-alpha

2. The process of Claim 1 wherein the temperature range for step (a) is in the range of about -30° to -80° C.

3. The process of Claim 1 wherein the temperature range for step (b) is about -80° to -40° C.

4. The process of Claim 1 wherein said sodium metal amide is sodium bis(trimethylsilyl)amide.

5. The process of Claim 1 wherein said solvent is a $C_2$-$C_{10}$-cyclic or acyclic ether.

6. The process of Claim 5 wherein said solvent is tetrahydrofuran.

7. The process of Claim 1 wherein said methylating agent is $CH_3I$.

The process of Claim 1 wherein said molar ratio of methylating agent to said structure I is about 2:1.

9. A process comprising the steps of:

(a) contacting the compound:

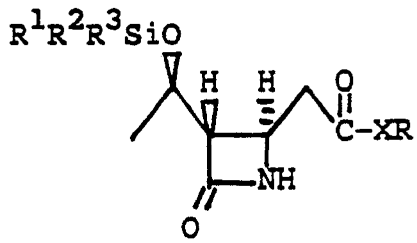

I

wherein X is -O- or -S-; R is $C_1$-$C_8$ linear or branched alkyl, $C_6$-$C_8$ aryl, or a carboxylic acid protecting group; and $R^1$, $R^2$, $R^3$ are selected from $C_1$-$C_8$ linear or branched alkyl, $C_6$-$C_8$ aryl; with a lithium metal amide in an anhydrous solvent therefor, excluding hexamethylphosphoramide, in an oxygen-free, moisture-free atmosphere;

(b) contacting the resulting mixture from step (a) with a tri-$C_1$-$C_6$ alkylaluminum;

(c) contacting the resulting mixture from step (b) with a methylating agent to produce a compound of the structure:

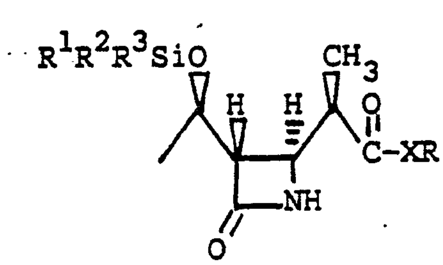

II-beta .

10. The process of Claim 9 wherein the temperature range for step (a) is in the range of about -30° to -85° C.

11. The process of Claim 9 wherein the temperature range for step (b) is about -40° to -80° C.

12. The process of Claim 9 wherein the temperature range for step (c) is -20° to -100° C.

13. The process of Claim 9 wherein said lithium metal amide is lithium diisopropylamide.

14. The process of Claim 9 wherein said solvent is a $C_2$-$C_{10}$-cyclic or acyclic ether.

15. The process of Claim 14 wherein said solvent is tetrahydrofuran.

16. The process of Claim 9 wherein step (b) said trialkylaluminum is selected from trimethylaluminum, triethylaluminum, tripropylaluminum, triisobutylaluminum or tri sec-butylaluminum.

17. The process of Claim 16 wherein said trialkylaluminum is triethylaluminum.

18. The process of Claim 9 wherein said methylating agent in step (c) is methyl iodide or methyl bromide.